(19) Europäisches Patentamt
European Patent Office
Office européen des brevets

(11) Numéro de publication: **0 267 076**
**A1**

(12) **DEMANDE DE BREVET EUROPEEN**

(21) Numéro de dépôt: **87402209.8**

(22) Date de dépôt: **06.10.87**

(51) Int. Cl.[4]: **C 08 G 65/48**
C 08 G 65/40, C 08 G 61/10

(30) Priorité: **09.10.86 FR 8614090**

(43) Date de publication de la demande:
**11.05.88 Bulletin 88/19**

(84) Etats contractants désignés:
**BE CH DE GB IT LI NL**

(71) Demandeur: **CENTRE D'ETUDE DES MATERIAUX ORGANIQUES POUR TECHNOLOGIES AVANCEES**
**Autoroute Lyon-Vienne Echangeur de Solaize Vernaison**
**Solaize**
**F-69390 Vernaison (FR)**

(72) Inventeur: **Dussart-Lermusiaux, Annie**
**1, rue du Tonkin**
**F-69100 Villeurbanne (FR)**

**Senneron, Michel**
**Résidence L'Emmendra 23, avenue du Vercors**
**F-38240 Meylan (FR)**

**Rabilloud, Guy**
**27, Boulevard Clémenceau**
**F-38100 Grenoble (FR)**

**Sillion, Bernard**
**93, rue Juliot Curie**
**F-69005 Lyon (FR)**

(74) Mandataire: **Colas des Francs, Jean**
**Institut Français du Pétrole 4, Avenue de Bois Préau**
**F-92502 Rueil-Malmaison (FR)**

(54) **Composition d'oligomères de polyaryloxypyridines à terminaisons acétyléniques, leur preparation, et les réseaux obtenus par leur polymérisation thermique.**

(57) On décrit des compositions d'oligomères de polyaryloxypyridine à terminaisons acétyléniques, obtenues par formation d'une composition d'oligomères de polyaryloxypyridine à terminaisons halogénées à partir d'un diphénolate alcalin et d'une dihalogénopyridine, suivie d'une réaction d'éthynylation, et pouvant présenter un degré moyen de polycondensation de 1 à 50. Elles peuvent être utilisées pour former des réseaux réticulés par réaction de polyaddition des groupements éthynyle.

EP 0 267 076 A1

## Description

La présente invention a pour objet de nouvelles compositions d'oligomères de polyaryloxypyridine terminés par des groupes fonctionnels acétyléniques. Elle concerne également la polymérisation thermique de ces compositions et les réseaux réticulés de polyaryloxypyridine qui sont formés au cours des réactions de polyaddition des groupements éthynyles.

Les compositions à base de polyaryloxypyridine à terminaisons acétyléniques de l'invention peuvent être employées comme liants dans la fabrication des matériaux composites, comme adhésifs, comme vernis de revêtement et comme matières premières pour faire des objets moulés et des matériaux cellulaires.

La recherche de nouveaux polymères thermostables thermodurcissables par des réactions d'addition qui ne dégagent pas de composés volatils au moment de la mise en oeuvre suscite beaucoup d'intérêt pour préparer des matériaux denses, homogènes et à faible porosité. Pour atteindre cet objectif, les réactions de polyaddition de groupes acétyléniques ont déja été utilisées pour réticuler des monomères ou des oligomères fusible et solubles. Ces réactions se produisent en effet par simple chauffage des réactifs au moment de leur mise en oeuvre avec formation de systèmes denses et réticulés.

De telles réactions d'addition ont été décrites avec des composés aromatiques, aliphatiques ou arylaliphatiques contenant des enchaînements amide, ester, éther, sulfone, cétone, imide, phénylène et quinoxaline. Ces réactions sont décrites par exemple dans les revues faites par P.M. Hergenrother, J. Macromol. Sci. Rev. Macromol. Chem., 1980, C19, 1, par C.Y. Lee, I.J. Goldfarb, F.E. Arnold, T.E. Helminiak, A.C.S. Polymer Preprints, 1982, 24(2), 904 et 28th Natl. SAMPE Symposium, 1983, 699, par A.O. Hanky, Natl. SAMPE Symposium, 1983, 711 et par B.A. Reinhardt, G.A. Loughran, F.E. Arnold, Polym. Sci. Technol., 1984, 25, 40. D'autres exemples de ces réactions sont donnés entre autres dans les brevets U.S. 4 022 746, 4 098 767, 4 100 138, 4, 131 625, 4 283 551 et dans le brevet Belge 898 889.

L'une des difficultés majeures rencontrée fréquemment avec les résines thermodurcissables basées sur les oligomères terminés par des fonctions acétyléniques, est que leur température de fusion ou de ramollissement est la plupart du temps supérieure à la température à laquelle il y a polymérisation des groupes acétyléniques. On a en effet observé que les réactions de polymérisation de ces groupes commencent à une température relativement basse, généralement inférieure à 200° C. Les techniques d'analyse thermique, analyse calorimétrique différentielle et analyse thermomécanique, montrent que la seuil thermique de la polymérisation est souvent situé entre 120° C et 180° C. Dans ces conditions, il est préférable, pour contrôler le processus de polymérisation, d'employer des compositions ayant une température de fusion ou de ramollissement assez largement inférieure à la température de début de polymérisation.

On sait que les polyéthers aromatiques, en particulier ceux dont les cycles aromatiques sont reliés entre eux par des atomes d'oxygène placés en position méta ont des températures de fusion assez basses puisque certains d'entre eux sont utilisés comme fluides thermostables. Mais la formation des liaisons éther entre des carbocycles aromatiques fait appel à des réactions de condensation qui se produisent à haute température et qui nécessitent la présence de systèmes catalytiques à base de cuivre.

On sait également que les atomes d'halogène placés en position ortho ou para d'un atome d'azote hétérocyclique sont beaucoup plus réactifs que ceux qui sont fixés sur les atomes de carbone d'un carbocycle. Parmi les composés hétérocycliques azotés qui ont une bonne résistance à la chaleur et à l'oxydation, la pyridine présente l'avantage d'être accessible industriellement. De ce fait, ses dérivés dihalogénés sur les positions 2 et 4 ou 2 et 6 constituent de bonnes matières premières pour synthétiser des oligomères de polyéthers aromatiques-hétérocycliques.

L'un des objets de la présente invention est de décrite de nouvelles compositions contenant des groupes acétyléniques, caractérisées par un temps de gel relativement court à une température de préférence inférieure à 250° C, des conditions de mise en oeuvre facilitées par rapport aux résines connues dans l'art antérieur et conduisant à des produits finis ayant de bonnes propriétés thermiques et mécaniques.

La présente invention concerne plus particulièrement la synthèse de nouvelles compositions d'oligomères de polyaryloxypyridine terminés par des groupes acétyléniques. Les résines de polyaryloxypyridine à terminaisons acétyléniques de l'invention présentent un intérêt exceptionnel car elles sont constituées de polyaryloxypyridine dont la température de fusion ou de ramollissement est pratiquement toujours inférieure à 200° C. Comme les enchaînements éther entre les cycles pyridine et les carbocycles automatiques sont en position méta sur la pyridine, les oligomères de polyaryloxypyridine de l'invention ont des propriétés de fusibilité, de solubilité et de souplesse tout à fait remarquables.

Un autre objet de l'invention concerne les produits réticulés obtenus au cours des réactions de polyaddition des groupes terminaux acétyléniques c'est à dire les réseaux formés au cours de la polymérisation thermique de ces groupes. Ces réseaux de caractérisent par une très bonne stabilité thermique et une bonne résistance à l'oxydation.

L'invention a plus particulièrement pour objet des compositions d'oligomères de polyaryloxypyridine terminés par des groupes acétyléniques que l'on peut représenter par la formule générale:

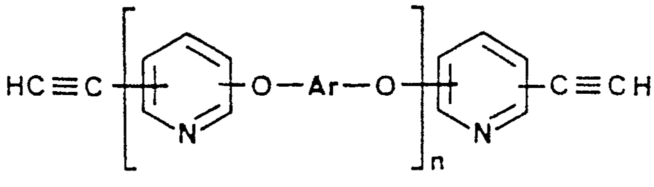 (1)

Dans cette formule, le radical Ar est un radical aromatique divalent, carbocyclique carbocyclique ou hétérocyclique, dont les deux valences sont sur des atomes de carbone distincts. Le radical Ar peut être formé d'un cycle ou de plusieurs cycles qui sont alors accolés ou reliés entre eux, chaque cycle étant formé de préférence de 5 à 7 atomes, dont une partie peut consister en atomes d'oxygène, de soufre et/ou d'azote.

Lorsque le radical Ar comporte plusieurs cycles reliés entre eux, les éléments de liaison sont par exemple la liaison simple ou l'un des atomes et groupements suivants:

-O-; -S-; -SO-; $-SO_2-$; $-CH_2-$; $-C(CH_3)_2-$; -CO-; -CHOH-; -COO-; -CONH-; $-Si(CH_3)_2-$; $-Si(CH_3)_2-O-Si(CH_3)_2-$.

Les éléments de liaison peuvent être également des radicaux divalents hydrocarbonés, partiellement ou totalement fluorés de type aliphatiques, arylaliphatiques ou cycloaliphatiques contenant de préférence de 1 à 10 atomes de carbone.

Les liaisons éthers entre le radical Ar et le cycle pyridine sont placées au milieu de ce dernier pour représenter l'existence d'isomères selon que les liaisons sont fixées sur l'un des atomes de carbone situés en position 2, 4 ou 6 de la pyridine.

n est un numbre qui indique le degré moyen de polycondensation. Il peut prendre par exemple une valeur de 1 à 50; le nombre n n'est pas toujours accessible directement, mais sa valeur moyenne se déduit des proportions molaires respectives des réactifs utilisés pour préparer les compositions d'oligomères de polyaryloxypyridine.

La méthode générale de synthèse des compositions de formule générale (1) consiste en une réaction d'éthynylation d'une composition d'oligomères de polyaryloxypyridine à terminaisons halogénées, que l'on peut représenter par la formule générale

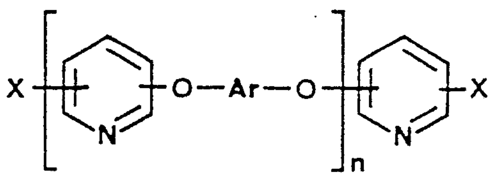 (2)

avec des composés porteurs d'une fonction acétylène de formules générales

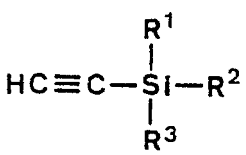 (3)

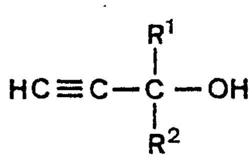 (4)

Dans ces formules, X représente un atome d'halogène, de préférence le brome ou le chlore, Ar a la signification indiquée précédemment, $R^1$, $R^2$ et $R^3$, identiques ou différents, sont des restes monovalents d'hydrocarbures aliphatiques ou aromatiques ayant de 1 à 13 atomes de carbone. Parmi les groupes qui conviennent plus particulièrement, on peut citer les groupes méthyle, éthyle, propyle, isopropyle, butyle, tertiobutyle, vinyle, isopropényle, phényle ou tolyle.

Les compositions d'oligomères de polyaryloxypyridine à terminaisons halogénées de formule générale (2) peuvent être préparées par réaction d'une dihalogéno-2,6 pyridine ou d'une dihalogéno-2,4 pyridine de formules:

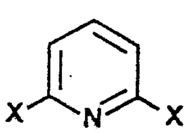 (5)

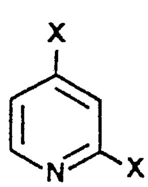 (6)

avec un défaut par rapport à la stoechiométrie moléculaire, d'au moins un diphénolate de formule générale:

M-O-Ar-O-M (7)

dérivé du diphénol correspondant.

Dans ces formules, X et Ar ont la signification indiquée précédemment et M représente un atome de métal alcalin, de préférence le sodium ou le potassium.

Parmi les diphénols qui conviennent plus particulièrement dans l'invention, on peut citre le dihydroxy-1,2 benzène, le dihydroxy-1,3 benzène, le dihydroxy-1,4 benzène, les dihydroxytoluènes, les dihydroxyxylènes, les dihydroxynaphthalènes, le dihydroxy-2,2′ biphényle, le dihydroxy-3,3′ biphényle, le dihydroxy-4,4′ biphényle, le bis(hydroxy-3 phényl) méthane, le bis(hydroxy-4 phényl) méthane, le bis(hydroxy-3 phényl) éther, le bis(hydroxy-4 phényl) éther, le bis(hydroxy-3 phényl) sulfure, le bis(hydroxy-4 phényl) sulfure, la bis(hydroxy-3 phényl) sulfone, la bis(hydroxy-4 phényl) sulfone, le bis(hydroxy-3 phényl) sulfoxyde, le bis(hydroxy-4 phényl) sulfoxyde, la dihydroxy-3,3′ benzophénone, la dihydroxy-4,4′ benzophénone, le bis(hydroxy-4 phényl)-2,2 propane, le bis(hydroxy-4 phényl)-2,2 hexafluoro-1,1,1,3,3,3 propane, le bis(hydroxy-3 phényl) diméthylsilane, le bis(hydroxy-4 phényl) diméthylsilane, le bis(hydroxy-3 phényl)-1,3-tétraméthyl-1,1,3,3-disiloxane, et le bis(hydroxy-4 phényl)-1,3 tétraméthyl-1,1,3,3-disiloxane.

Parmi les dihalogénopyridines utilisables plus particulièrement dans le cadre de la présente invention, on peut citer la difluoro-2,6 pyridine, la dichloro-2,6 pyridine, la dibromo-2,6 pyridine, la diiodo-2,6 pyridine, la difluoro-2,4 pyridine, la dichloro-2,4 pyridine, la dibromo-2,4 pyridine et la diiodo-2,4 pyridine.

Les compositions de formule générale (1) son préparées en deux étapes principales consécutives avec isolement des composés intermédiaires qui sont respectivement:a) - la préparation d'oligomères de polyaryloxypyridine à terminaisons halogénées de formule (2); et b) - la réaction d'éthynylation desdites compositions qui consiste à substituer les atomes d'halogène par un composé acétylénique dont l'une des sites réactifs est protégé par un groupe protecteur, cette réaction étant effectuée en présence d'un système catalytique, puis à débloquer la fonction acétylénique bloquée.

Dans la première étape, les compositions de polyaryloxypyridine à terminaisons halogénées (2) sont elles-mêmes préparées en deux réactions successives avec ou sans isolement des composés intermédiaires.

Dans la première réaction, un diphénol est transformé en diphénolate alcalin de formule (7) par réaction avec un métal alcalin, un hydroxyde, un carbonate ou un alcoolate de métal alcalin. Cette réaction est effectuée selon des méthodes bien connues, de préférence en solution dans un solvant organique polaire auquel on peut ajouter un hydrocarbure aliphatique ou aromatique qui permet d'éliminer l'eau de la réaction, lorsqu'il y en a, par distillation azéotropique. Dans tous les cas, il est préférable de maintenir le milieu réactionnel à l'abri de l'humidité.

Dans la seconde réaction, la dihalogénopyridine de formule (5) ou (6) est ajoutée au diphénolate (7), dans des proportions moléculaires telles qu'il y ait toujours un défaut de ce dernier réactif. Ainsi la proportion molaire du diphénolate (7) à la dihalogénopyridine (5) ou (6) est en général d'environ 1/1,02 à 1/2, de manière que l'on obtienne une composition d'oligomères de polyaryloxypyridine à terminaisons halogénées de formule générale (2) avec un degré moyen de polycondensation convenable (n de 1 à 50). Cette réaction de condensation qui conduit à la formation d'enchaînements éther est effectuée par chauffage entre 50 et 300° C, de préférence entre 90 et 250° C. Elle peut être effectuée à la fusion des réactifs, mais il est préférable d'employer un solvant organique polaire comme par exemple 1a N-méthyl pyrrolidone, le diméthylsulfoxyde, le diméthylacétamide ou le diméthylformamide.

La deuxième étape consiste en une réaction de subsitution des groupes halogènes terminaux des compositions de polyaryloxypyridine (2) par des composés acétyléniques dont l'un des sites réactifs est protégé par un groupe protecteur. lesdits composés étant représentés par les formules générales (3) et (4 ), suivi de l'élimination de ce groupe protecteur.

La réaction des polyaryloxypyridines à terminaisons halogénées (2) avec les composés acétyléniques (3) ou (4) est effectuée de préférence en solution dans un solvant organique, en présence d'un composé basique susceptible de fixer les atomes d'halogènes terminaux qui sont éliminés sous forme d'hydracide et d'un systène catalytique comprenant des composés du cuivre et du palladium et, éventuellement, un agent de coordination. Cette réaction conduit à des polyaryloxypyridines de formules générales:

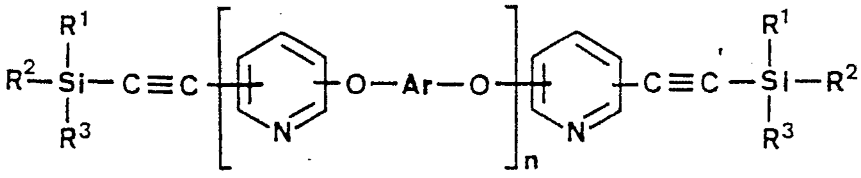

(8)

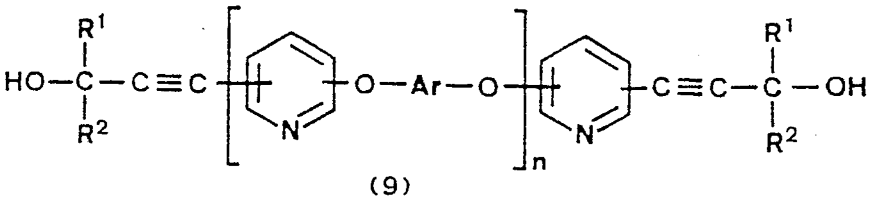

(9)

La réaction de substitution des atomes d'halogène par les composés acétyléniques (4) ou (5) est effectuée par chauffage entre 20° C et 200° C, de préférence de 50° C à 120° C, en solution dans un solvant qui peut être un solvant organique inerte vis à vis des produits en réaction, mais qui peut être également le composé basique utilisé pour fixer l'hydracide.

Les polyaryloxypyridines à terminaisons acétyléniques de formule (1) sont alors obtenues par désilylation des compositions de formule (8) ou par élimination d'une cétone de formule $R^1$-CO-$R^2$ dans les compositions de formule (9)

Parmi les composés du palladium utilisables avantageusement dans le système catalytique, on peut citer les composés de formules générales $Pd(PR_3)_4$, $Pd(PR_3)_2X_2$, $Pd(O_2CR)_2$, $Pd(O_2CR)_2(PR_3)_2$ et $Pd(AsR_3)_2X_2$ dans lesquelles X est un atome d'halogène et R représente un groupe alkyle, aralkyle ou aryle. Comme exemple de ces composés, on peut citer l'acétate de palladium, le diacétato bis(triphénylphosphine) palladium, le dichloro bis(triphénylphosphine) palladium, le tétrakis(triphénylphosphine) palladium, le bis(bis(diphénylphosphino)-1,2-éthane) palladium et le dichloro bis(triphénylarsino) palladium.

Parmi les composés du cuivre utilisables dans le système catalytique, on peut citer le chlorure cuivreux, l'iodure cuivreux, le bromure cuivreux, l'oxyde cuivreux et le cyanure cuivreux.

Les agents de coordination que l'on peut ajouter au système catalytique sont généralement des dérivés du phosphore, de l'arsenic ou de l'antimoine, comme par exemple, la triphénylphosphine, la tris(ortho-tolyl) phosphine, la triphénylarsine et la triphénylstibine.

Les composés basiques qui servent, entre autre, à capter les molécules d'hydracide libérées au cours de la réaction sont de préférence des composés aminés parmi lesquels on peut citer les amines primaires comme l'hexylamine, la benzylamine et l'aniline, les amines secondaires comme la diméthylamine, la diéthylamine, la méthyléthylamine, la N-méthylaniline, la N-éthylaniline et la pipéridine et les amines tertiaires comme la triméthylamine, la triéthylamine, la N,N-diméthylaniline, la benzyldiméthylamine, et la N,N,N',N'-tétraméthyl éthylènediamine.

Les amines ayant une température d'ébullition comprise entre 50° C et 150° C comme la diéthylamine, la triéthylamine et la N,N,N',N'-tétraméthyléthylènediamine sont particulièrement bien adaptées.

La substitution des atomes d'halogène des polyaryloxypyridines (2) par les composés acétyléniques (3) ou (4) peut être effectuée en solution dans l'un des composés aminés cités ci-dessus, mais il est également possible d'ajouter au mileiu réactionnel un autre solvant qui peut être un hydrocarbure comme par exemple l'hexane ou le benzène, un hydrocarbure halogéné comme la dichlorométhane ou le chloroforme, ou un éther comme l'éther de diéthyle, l'éther de dibutyle, le tétrahydrofuranne, l'acétate d'éthyle ou le dioxanne.

La réaction de désilylation des polyaryloxypyridines de formule (8) est effectuée en solution dans un mono-alcool aliphatique, comme le méthanol, l'éthanol l'isopropanol ou le tertiobutanol en présence d'une base minérale comme l'hydroxyde de sodium, l'hydroxyde de potassium, le carbonate de potassium ou le fluorure de potassium, à une température de préférence comprise entre 50° C et 150° C et de préférence en atmosphère inerte. Cette réaction de désilylation peut être faite en utilisant uniquement comme solvant le mono-alcool mail il est également possible d'ajouter au milieu réactionnel un co-solvant inerte comme l'éther de diéthyle, le tétrahydrofuranne ou le dioxanne.

La réaction d'élimination des cétones sur les polyaryloxypyridines de formule (9) est effectuée en solution dans un solvant organique qui peut être un hydrocarbure, un éther, une amine, un aldéhyde, un alcool ou un ester, en présence d'une base minérale comme l'hydroxyde de sodium ou l'hydroxyde de potassium à une température comprise entre 50° C et 150° C et de préférence en milieu anhydre et en atmosphère inerte.

Les compositions à base de polyaryloxypyridine à terminaisons acétyléniques de l'invention sont des compositions thermodurcissables. Le processus réactionnel qui permet de transformer, par chauffage, les oligomères fusibles et solubles en systèmes denses et réticulés n'est par une réaction simple. Il y a

superposition de plusieurs réactions de polymérisation et de cyclotrimérisation qui conduisent finalement au système réticulé.

D'une façon générale, les compositions de l'invention sont polymérisées par chauffage à une température comprise entre 100° C et 250° C, pendant un temps qui varie de quelques minutes à quelques heures. Après la phase de polymérisation, le degré de réticulation peut encore être augmenté par un traitement thermique complémentaire à plus haute température, par exemple de 200° C à 300° C. Cette opération permet de poursuivre en phase solide les réactions de polymérisation et d'accroître la température de transition vitreuse des polymères de plusieurs dizaines de degrés centigrades.

Les polymères réticulés obtenus par polymérisation thermique des compositions de polyaryloxypyridine à terminaisons acétyléniques se caractérisent par une excellente tenue à la chaleur et à l'oxydation. Ainsi lorsque la réaction de polymérisation est conduite à 180° C pendant deux heures, l'analyse thermogravimétrique isotherme à 300° C pendant 20 heures montre que la perte de poids des systèmes les plus fortement réticulés est inférieure à 3%.

On inclut dans l'invention les compositions d'oligomères de polyaryloxypyridines à terminaisons acétyléniques qui répondent à la formule générale (1) donnée plus haut mais dans lesquelles les enchaînements éther sont situés sur les atomes de carbone en position 3 et 5 des noyaux pyridine. En effet, on a découvert que si l'effet d'activation des atomes d'halogène placés en position ortho ou para est plus élevé que sur les sommets placés en position méta, la présence de l'atome d'azote dans l'hétérocycle facilite nettement les réactions de substitution nucléophile sur ces derniers sommets si on les compare aux hydrocarbures aromatiques carbocycliques halogénés. En utilisant des conditions expérimentales adaptées à cette différence de réactivité, il est donc possible de préparer des compositions de polyaryloxypyridines à terminaisons halogénées en faisant réagir un diphénolate alcalin avec une dihalogéno-3,5 pyridine.

Le processus général de synthèse de ces compositions est identique à celui qui est décrit précédemment. Il consiste à préparer d'abord des compositions de polyaryloxypyuridines à terminaisons halogénées en faisant réagir une dihalogéno-3,5 pyridine avec un défaut par rapport à la stoechiométrie moléculaire d'au moins un diphénolate alcalin.

Les dihalogéno-3,5 pyridines utilisables sont les difluoro-, dichloro-, dibromo- et diiodo-3,5 pyridines.

La deuxième étape consiste en une réaction de substitution des groupes halogènes terminaux desdites compositions de polyaryloxypyridines à terminaisons halogénées par des composés acétyléniques dont l'un des sites réactifs est protégé par un groupe protecteur, suivie de l'élimination de ce groupe protecteur. Toutes ces réactions sont décrites plus haut.

On a observé que les compositions de polyaryloxypyridines à terminaisons acétyléniques, issues de la mise en jeu de dihalogéno-3,5 pyridines, présentent sur certains points des caractéristiques améliorées par rapport aux compositions dérivant des dihalogéno-2,4 et 2,6 pyridines. En particulier, elles ont généralement une température de fusion ou de ramolissement plus basse et une plus grande stabilité thermique. Elles conviendront donc mieux pour des applications qui exigent une mise en oeuvre initiale à plus basse température, tout en nécessitant une très bonne stabilité thermique du réseau final.

L'invention sera décrite de façon plus précise en liaison avec les exemples spécifiques ci-après dans lesquels les détails sont donnés à titre illustratif et non limitatif. Dans ces exemples, les réactions de polycondensation sont effectuées sous agitation et en atmosphère inerte d'azote ou d'argon pour éviter toute réaction d'oxydation. Les produits obtenus sont caractérisés par l'analyse élémentaire, la spectroscopie infrarouge, la résonance magnétique nucléaire du proton et du carbone-13, l'analyse enthalpique différentielle, la chromatographie d'exclusion stérique et la chromatographie sur gel perméable. Le degré de polycondensation des polyaryloxypyridines est déterminé par chromatographie sur gel perméable, par le dosage des groupes halogène terminaux (dosage qui est exprimé en poids d'halogène dans 100 grammes de produit), ainsi que par résonance magnétique nucléaire.

Exemple 1

Un mélange de 59,2 grammes (0,4 mole) de dichloro-2,6 pyridine, 22 grammes (0,2 mole) de dihydroxy-1,3 benzène, 41,49 grammes de carbonate de potassium, 110 $cm^3$ de N-méthyl pyrrolidinone -2 et 75 $cm^3$ de toluène est chauffé pendant 7 heures à 130° C avec élimination, par distillation azéotropique, de l'eau formée au cours de la réaction. Le toluène est ensuite distillé et la solution est versée dans un litre d'eau. Le produit qui précipite est lavé à l'eau, filtré et séché à 50° C sous pression réduite.

Le produit obtenu avec un rendement de 90% (59,9 g) a une température de fusion de 81° C. L'analyse de ce composé, dont la masse moléculaire est de 333 grammes, permet de l'identifier au bis(chloro-2 pyridyl-6 oxy)-1,3 benzène répondant à la formule:

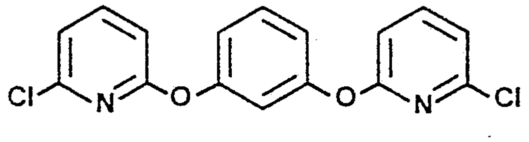

Exemples 2 à 8

Une série de polyaryloxypyridines terminées par des fonctions halogénées est préparée en utilisant les conditions expérimentales de l'exemple 1 avec les réactifs indiqués dans le tableau 1.

TABLEAU 1

| Exemple n° | Diphénol[1)] | Dihalogéno pyridine | Rendement (%) |
|---|---|---|---|
| 2 | R | Dibromo-2,6 | 91 |
| 3 | A | Dichloro-2,6 | 96 |
| 4 | A | Dibromo-2,6 | 94 |
| 5 | F | Dibromo-2,6 | 63 |
| 6 | S | Dibromo-2,6 | 90 |
| 7 | A | Dibromo-2,4 | 89 |
| 8 | A | Dichlroro-2,4 | 95 |

1) Les diphénols utilisés sont les suivants :

A : Bis(hydroxy-4 phényl)-2,2 propane

F : Bis(hydroxy-4 phényl)-2,2 hexafluoro-1,1,1,3,3,3 propane

S : Bis(hydroxy-4 phényl) sulfure

R : Dihydroxy-1,3 benzène (résorcinol)

Les quantités employées correspondent à 0,4 mole de dihalogénopyridine, 0,2 mole de diphénol, 0,3 mole de carbonate de potassium, 110 cm³ de N-méthyl pyrrolidinone-2 et 75 cm³ de toluène. Les analyses chimiques et spectroscopiques des produits formés dans ces réactions permettent de les identifier respectivement aux composés suivants.

Exemple 2 : Bis(bromo-2 pyridyl-6 oxy)-1,3 benzène ayant une température de fusion de 93° C et une masse moléculaire de 422 grammes. Ce composé peut être représenté par la formule chimique suivante:

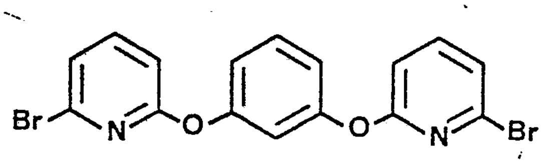

Exemple 3 : Bis((chloro-2 pyridyl-6 oxy)-4-phényl)-2,2 propane ayant un point de fusion de 142°C et une masse moléculaire de 451 grammes et répondant à la formule suivante:

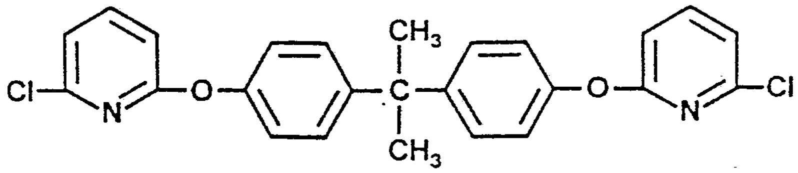

Exemple 4 : Bis((bromo-2 pyridyl-6 oxy)-4 phényl)-2,2 propane ayant un point de fusion de 127°C et une masse moléculaire de 540 grammes dont la formule moléculaire est la suivante:

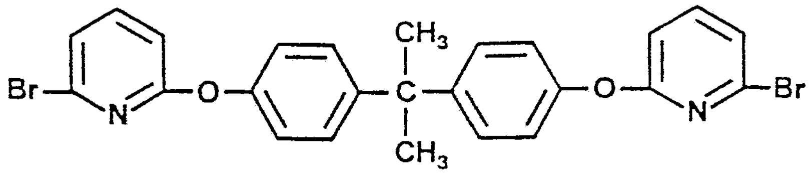

Exemple 5 : Bis((bromo-2 pyridyl-6 oxy)-4 phényl)-2,2 hexafluoro-1,1,1,3,3,3 propane ayant une température de fusion de 120°C, une masse moléculaire de 648 grammes et qui répond à la formule suivante:

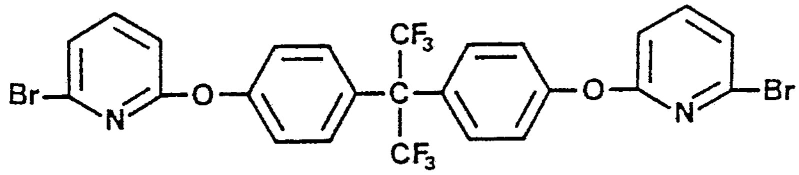

Exemple 6 : Bis((bromo-2 pyridyl-6 oxy)-4 phényl) sulfure ayant une température de fusion de 82°C et une masse moléculaire de 530 grammes.Ce composé est représenté par la formule suivante:

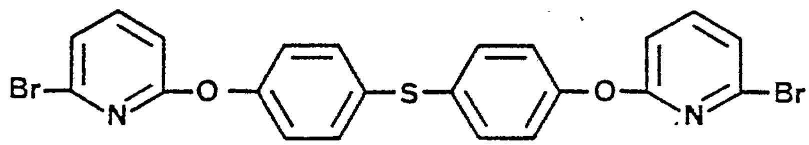

Exemple 7 : L'analyse chromatographique montre que le produit obtenu est un mélange de trois composés qui sont probablement le bis((bromo-2 pyridyl-4 oxy)-4 phényl)-2,2 propane, le bis((bromo-4 pyridyl-2 oxy)-4 phényl)-2,2 propane et le ((bromo-2 pyridyl-4 oxy)-4 phényl (bromo-4 pyridyl-2 oxy)-4' phényl)-2,2 propane.

Exemple 8 : Comme dans l'exemple 7, l'analyse chromatographique montre que le produit obtenu est également un mélange de deux ou trois isomères.

Exemple 9

Un mélange de 11,4 grammes (0,05 mole) de bis(hydroxy-4 phényl)-2,2 propane, 4 grammes (0,1 mole) d'hydroxyde de sodium, 17 $cm^3$ de diméthylsulfoxyde et 10 $cm^3$ de toluène est chauffé pendant 4 heures à 120-130°C, avec élimination de l'eau formée au cours de la réaction par distillation azéotropique du mélange eau-toluène. Le toluène est ensuite distillé en totalité avant addition de 11,1 grammes (0,075 mole) de dichloro-2,6 pyridine. La réaction d'éthérification est effectuée par chauffage du milieu réactionnel pendant 4 heures à 150°C. Le mélange refroidi est versé dans une solution aqueuse normale d'hydroxyde de sodium. Le précipité qui s'est formé est isolé par filtration, lavé à l'eau et séché à poids constant à 50°C sous pression réduite. Le produit obtenu avec un rendement de 97% peut être identifié à une polyaryloxypyridine de formule:

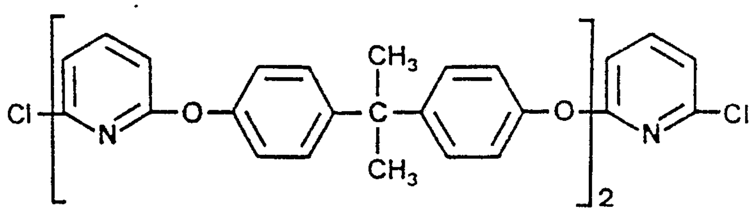

Exemples 10 à 15

Une série de polyaryloxypyridines terminées par des fonctions halogénées, ayant un degré de polycondensation ($\overline{DPn}$) compris entre 2 et 50, est préparée dansles conditions expérimentales de l'exemple 9. Le poids en grammes de dihalogénopyridine, indiqué dans le tableau 2, est mis en réaction avec 13,7 grammes (0,06 mole) de bis(hydroxy-4 phényl)-2,2 propane et 4,8 grammes (0,12 mole) de soude.

TABLEAU 2

| Exemple n° | dichloro-2,6 pyridine | dibromo-2,6 pyridine | $\overline{DPn}$ Calc[1)] | $\overline{DPn}$ Trouvé[2)] |
|---|---|---|---|---|
| 10 | | 18,95 | 3 | 2,5 |
| 11 | 10,66 | | 5 | 2,5 |
| 12 | | 16,58 | 6 | 5,92 |
| 13 | 9,77 | | 10 | 3,6 |
| 14 | | 15,16 | 15 | 9,2 |
| 15 | | 14,50 | 50 | 20,3 |

1)Calculé à partir des proportions respectives de dihalogénopyridine et de diphénol.

2)Trouvé par l'analyse élémentaire quantitative des atomes d'halogène.

Selon que le produit de départ est la dibromo-2,6 pyridine ou la dichloro-2,6 pyridine, les polymères préparés dans les exemples 10 à 15 répondent à la formule suivante dans laquelle X est l'atome d'halogène (brome ou chlore) et n prend la valeur indiquée pour le $\overline{DPn}$ dans le tableau 2.

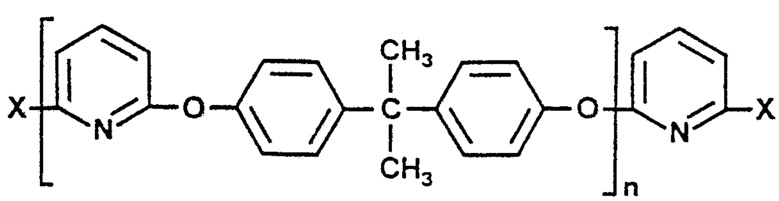

Exemple 16

Un mélange de 10,57 grammes (0,025 mole) de bis(bromo-2 pyridyl-6 oxy)-1,3 benzène préparé dans l'exemple 2, 5,04 grammes (0,06 mole) de méthyl-2 butyn-3-ol-2, 0,075 gramme (0,1 mmole) de bis(triphénylphosphine) dichloropalladium, 0,4 gramme (1,5 mmole) de triphénylphosphine, 0,075 gramme (0,395 mmole) d'iodure cuivreux et 50 $cm^3$ de triéthylamine est chauffé pendant 6 heures à 110°C en atmosphère d'argon. Le mélange refroidi est filtré pour éliminer le chlorhydrate de triéthylamine et le solvant est évaporé sous pression réduite. Le résidu est dissous dans de l'éther éthylique et cette solution est lavée plusieurs fois avec de l'eau distillée. L'évaporation de l'éther donne avec un rendement de 66% un produit qui peut être identifié au bis((hydroxy-3 méthyl-3 butynyl)-2 pyridyl-6 oxy)-1,3 benzène ayant un point de fusion de 143°C et représenté par la formule:

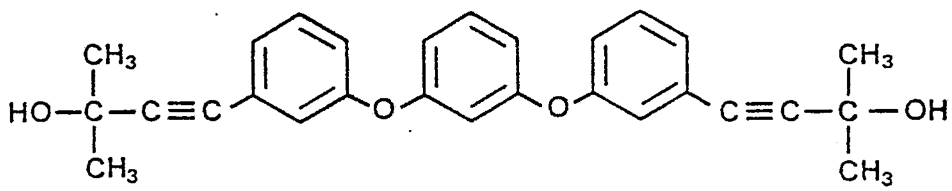

Une solution de 3,21 grammes (7,5 mmole) de ce composé dans 35 $cm^3$ de toluène anhydre est chauffé au reflux du solvant, en présence de 0,5 gramme d'hydroxyde de sodium, pendant 2 heures. La solution est refroidie, filtrée et le toluène est distillé sous pression réduite. Le résidu est dissous dans l'éther, lavé plusieurs fois à l'eau pour donner, après évaporation de l'éther, un produit solide dont la température de fusion est de 100°C. Ce composé qui est obtenu avec un rendement de 50% est identifié au bis(éthynyl-2 pyridyl-6 oxy)-1,3 benzène de formule:

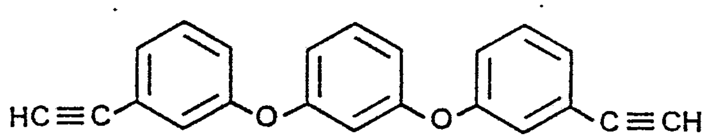

Exemple 17

Les conditions expérimentales de l'exemple 16 sont utilisées pour préparer le bis((éthynyl-2 pyridyl-6 oxy)-4 phényl)-2,2 propane à partir du composé halogéné décrit dans l'exemple 3.

Le composé obtenu a une température de fusion de 103°C et une masse moléculaire de 430 grammes par mole et il répond à la formule suivante:

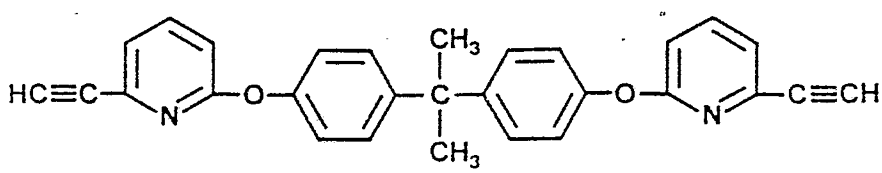

Exemple 18

Un mélange de 16,88 grammes (0,04 mole) de bis(bromo-2 pyridyl-6 oxy)-1,3 benzène, obtenu comme décrit dans l'exemple 2, 10 grammes (0,1 mole) d'éthynyltriméthylsilane, 0,1 gramme (0,14 mmole) de bis(triphénylphosphine)-dichloropalladium, 0,3 gramme (1,15 mmole) de triphénylphosphine, 0,1 gramme (0,5 mmole) d'iodure cuivreux et 100 $cm^3$ de triéthylamine est chauffé pendant 7 heures à 50°C en autoclave. Le mélange refroidi est filtré et la triéthylamine est distillée sous pression réduite.

Le résidu est dissous dans l'éther, lavé à l'eau et l'éther est évaporé pour donner un produit brut qui est recristallisé dans l'heptane. Le rendement en produit pur est de 70%. Le composé obtenu a une température de fusion de 107°C et il est identifié au bis((triméthylsilyl-2 éthynyl)-2 pyridyl-6 oxy)-1,3 benzène répondant à la formule

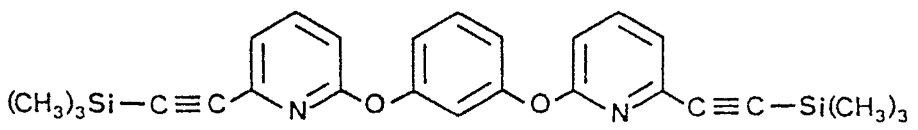

Une solution de 2,28 grammes (5 mmole) de ce composé dans 10 $cm^3$ de méthanol est agitée à la température ambiante avec 0,56 gramme (0,01 mole) d'hydroxyde de potassium pendant une heure. Après addition d'une solution aqueuse d'acide chlorhydrique 3N (5 $cm^3$), la solution est concentrée sous pression réduite, reprise à l'éther éthylique et lavée plusieurs fois à l'eau. L'éther est évaporé pour donner un produit brut qui est recristallisé dans le cyclohexane. Le rendement en produit pur est de 50% et ses caractéristiques sont identiques à celles qui sont décrites dans l'exemple 16.

Exemples 19 à 21

Les conditions expérimentales de l'exemple 18 sont employées pour préparer des polyaryloxypyridines à terminaisons acétyléniques à partir des composés halogénés décrits dans les exemples 4, 5 et 6. L'analyse des produits obtenus dans ces réactions donne les résultats suivants.

Exemple 19 : Bis((éthynyl-2 pyridyl-6 oxy)-4 phényl)-2,2 propane identique au composé préparé dans l'exemple 17.

Exemple 20 : Bis((éthynyl-2 pyridyl-6 oxy)-4 phényl)-2,2 hexafluoro-1,1,1,3,3,3 propane ayant une masse moléculaire de 538 grammes par mole, une température de fusion de 129°C et qui répond à la formule suivante:

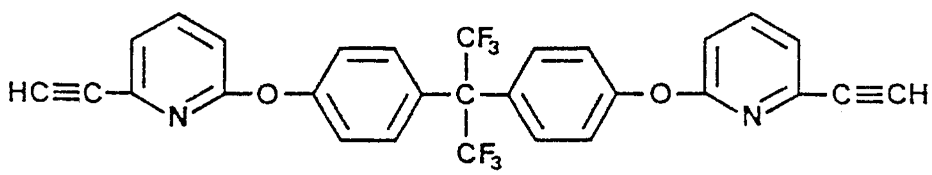

Exemple 21 : Bis((éthynyl-2 pyridyl-6 oxy)-4 phényl) sulfure dont la température de fusion est de 97°C, la masse moléculaire de 420 grammes par mole et qui peut être représenté par la formule:

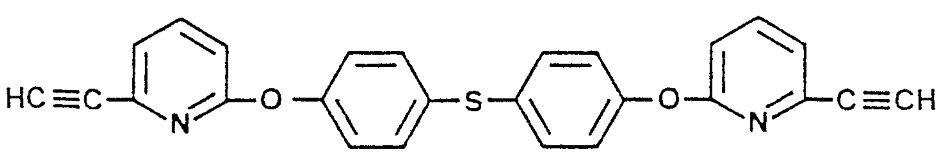

Exemple 22

Un mélange de 7 grammes de polyaryloxypyridine dibromée comme obtenu dans l'exemple 12, 0,67 gramme (6,86 mmole) d'éthynyltriméthylsilane, 0,0092 gramme (0,0131 mmole) de bis(triphénylphosphine) dichloropalladium, 0,0092 gramme (0,048 mmole) d'iodure cuivreux, 0,0276 gramme (0,105 mmole) de triphénylphosphine, 10 $cm^3$ de triéthylamine et 40 $cm^3$ d'acétate d'éthyle est chauffé pendant 24 heures à 50°C en autoclave.

Le mélange refroidi est filtré et les solvants sont distillés sous pression réduite. Le résidu est broyé dans du méthanol, filtré et séché pour donner un produit dont le point de fusion est de 86°C et qui peut être représenté par la formule:

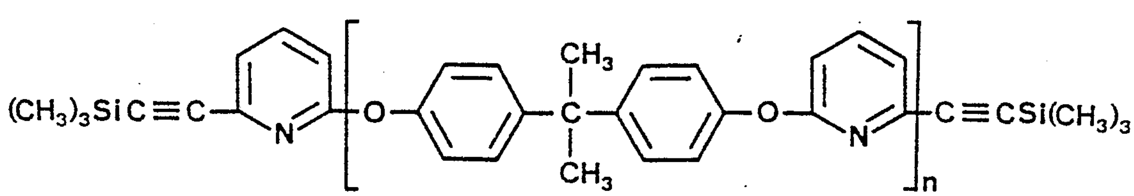

Une solution de 2 grammes de ce produit dans un mélange de 3 $cm^3$ de méthanol et 2 $cm^3$ de dioxanne est agité à température ambiante avec 0,1 gramme d'hydroxyde de potassium, pendant une heure sous

atmosphère inerte. Après addition de 1 $cm^3$ d'une solution aqueuse d'acide chlorhydrique 3N, le mélange est concentré sous pression réduite, puis redissout dans de l'acétate d'éthyle et lavé plusieurs fois à l'eau

La phase organique est séparée, séchée et amenée à sec par évaporation du solvant. Le résidu est broyé dans du méthanol, filtré et séché. Le produit obtenu a une température de fusion de 71°C, une masse moléculaire, mesurée par chromatographie sur gel perméable, de 1748 grammes par mole et il peut être représenté par la formule suivante d'un (éthynyl pyridylène-2,6) poly(oxyphénylène-1,4-(méthyl-1 éthylidène)-phénylèneoxy-1,4-pyridylène-2,6)-éthynyle dans laquelle n est à peu près égal à 5,4.

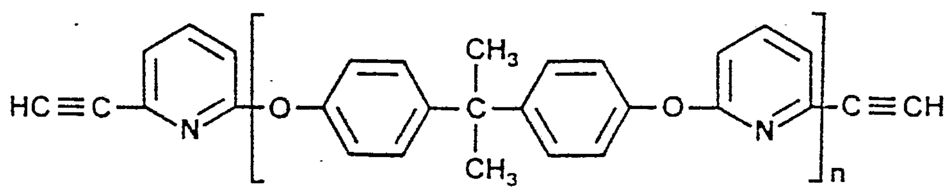

Exemples 23 à 25

Les conditions expérimentales de l'exemple 22 sont utilisées pour préparer des oligomères répondant à la formule indiquée ci-dessus pour un (éthynyl pyridylène-2,6) poly(oxyphénylène-1,4-(méthyl-1 éthylidène)-phénylèneoxy-1,4-pyridylène-2,6)-éthynyle en partant des composés dihalogénés décrits dans les exemples 10, 14 et 15. L'analyse des produits obtenus après fixation du triméthylsilylacétylène et désilylation en milieu basique indique que les polymères ont les caractéristiques suivantes.

Exemple 23 : n = 2,5 température de fusion = 83°C; masse moléculaire moyenne comprise entre 890 à 1000 grammes par mole.

Exemple 24 : n = 9; température de ramollissement = 69°C; masse moléculaire moyenne comprise 2900 à 3500 grammes par mole.

Exemple 25 : n = 20; température de ramollissement = 80-110°C; masse moléculaire moyenne comprise entre 6200 et 8400 grammes par mole.

Exemple 26

Le bis(éthynyl-2 pyridyl-6 oxy)-1,3 benzène préparé dans l'exemple 16 est étudié avec un appareil d'analyse calorimétrique différentielle programmé pour une montée en température de 10°C par minute.

Le thermogramme obtenu lors du premier passage présente un pic endothermique de fusion à 100°C et un pic exothermique de polymérisation qui commence vers 115°C et se termine vers 260°C.

Lorsque le produit est maintenu pendant 2 heures à 180°C puis ramené à la température ambiante, le thermogramme obtenu en analyse calorimétrique différentielle montre que les pics de fusion et de polymérisation ont pratiquement disparu et au second passage il n'apparait aucune variation de pente qui correspondrait à une température de transition vitreuse.

Le matériau obtenu après deux heures de polymérisation à 180°C est examiné en analyse thermogravimétrique dynamique avec des vitesses de montée en température de 1 et 5°C par minute. Cette méthode permet de déterminer la stabilité thermique du produit avec plus de précision car le seuil de dégradation thermique varie en fonction du programme de montée en température.

Dans le premier cas, les seuils de décomposition sont situés à 356°C à l'air et à 379°C sous argon. Dans le second cas, la décomposition commence à 402°C à l'air et en atmosphère inerte.

Lorsque le matériau est étudié en analyse thermogravimétrique isotherme à 300°C à l'air, la perte du poids est de 2,7% après 20 heures de traitement.

Exemples 27 à 29

Les analyses thermiques décrites dans l'exemple 26 sont réalisées sur le bis((éthynyl-2 pyridyl-6 oxy)-4 phényl)-2,2 propane de l'exemple 19, sur le bis((éthynyl-2 pyridyl-6 oxy)-4 phényl)-2,2 hexafluoro-1,1,1,3,3,3 propane de l'exemple 20 et sur le bis((éthynyl-2 pyridyl-6 oxy)-4 phényl) sulfure de l'exemple 21. Les résultats obtenus sont les suivants.

| Exemple n° | 27 | 28 | 29 |
|---|---|---|---|
| Résine de l'exemple | 19 | 20 | 21 |
| Température de fusion | 103°C | 129°C | 97°C |
| Début de polymérisation | 130°C | 130°C | 135°C |
| Fin de polymérisation | 295°C | 305°C | 290°C |
| Transition vitreuse | néant | néant | néant |
| Seuil de décomposition à l'air | 385°C | 373°C | 345°C |
| Seuil de décomposition sous argon | 390°C | 394°C | 360°C |

Exemples 30 à 32

Les analyses thermiques sont effectuées sur les oligomères de plus forte masse moléculaire dont la synthèse est décrite dans les exemples 22, 23 et 24 avec les résultats suivants.

| Exemple n° | 30 | 31 | 32 |
|---|---|---|---|
| Résine de l'exemple | 22 | 23 | 24 |
| DPn de la résine | 5,4 | 2,5 | 9 |
| Début de polymérisation | 135°C | 130°C | 135°C |
| Fin de polymérisation | 290°C | 285°C | 290°C |
| Transition vitreuse : | | | |
| - après 2 heures à 180°C | 94°C | 116°C | 88°C |
| - après 16 heures à 250°C | 113°C | 140°C | 109°C |
| Seuil de décomposition à l'air | 297°C | 344°C | 295°C |
| Seuil de décomposition sous argon | 297°C | 365°C | 295°C |

Exemple 33

Un mélange de 14,22 grammes (0,060 mole) de dibromo-3,5 pyridine, 4,56 grammes (0,020 mole) de bis(hydroxy-4 phényl)-2,2 propane, 6,35 grammes de carbonate de potassium, 0,22 gramme de chlorure cuivreux, 0,16 gramme d'iodure de potassium, 80 cm³ de N-méthyl pyrrolidinone-2 et 40 cm³ de toluène est chauffé à 144° C avec élimination azéotropique de l'eau formée au cours de la réaction. La durée du chauffage, qui est de 8,5 heures, est fixée par la disparition complète des groupements phénol. Après refroidissement, le mélange réactionnel est filtré et le filtrat est concentré par distillation sous pression réduite de la majeure partie des solvants. Le résidu est versé dans de l'eau et le précipité qui se forme est lavé à l'eau et séché. Il est ensuite repris dans 50 cm³ d'acétate d'éthyle. La solution est filtrée pour éliminer les impuretés insolubles dans l'acétate d'éthyle. Après évaporation du solvant, le résidu est extrait avec 250 cm³ d'hexane bouillant. Cette opération donne deux fractions. La première est soluble dans l'hexane alors que la seconde donne une phase non miscible.

La fraction soluble dans l'hexane est isolée par évaporation du solvant et séchage prolongé à 100° C sous pression réduite. Le produit isolé sous forme d'une huile jaune clair pèse 4,38 grammes (rendement de 41%) et il répond à la formule:

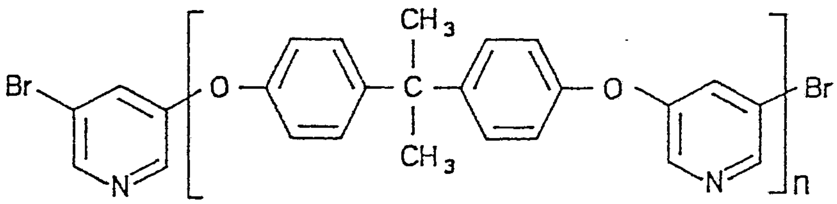

L'analyse de ce produit par chromatographie sur gel perméable montre qu'il est constitué d'oligomères répondant à la formule précédente avec 84% du composé pour lequel le degré de polycondensation n est égal à 1, 11% de l'oligomère pour lequel n est égal à 2 et 5% d'un composé non identifié ayant une masse moléculaire apparente de 370 grammes.mole$^{-1}$.

La fraction insoluble dans l'hexane se présente comme une huile visqueuse brune dont le poids est de 3,11 grammes. Les analyses chimiques, spectroscopiques et chromatographiques indiquent qu'elle est formée d'oligomères répondant à la formule précédente avec la répartition suivante : 29% d'oligomère n = 1, 34% d'oligomère n =2, 20% d'oligomère n = 3 et 17% d'oligomères supérieurs.

Exemple 34

Un mélange de 8,66 grammes de la première fraction préparée dans l'exemple 33 et qui est principalement constituée de bis((bromo-3 pyridyl-5 oxy)-4 phényl)-2,2 propane correspondant à 31,7 milliéquivalents de brome, 3,3 grammes (0,0396 mole) de méthyl-2 butyn-3-ol-2, 44 milligrammes de bis(triphénylphosphine) dichloropalladium, 0,249 gramme de triphénylphosphine, 46 milligrammes d'iodure cuivreux et 60 cm$^3$ de triéthylamine est chauffé pendant 5 heures à 110° C en atmosphère d'azote. Après refroidissement, le mélange est filtré et le précipité est rincé avec de l'éther éthylique. Les fractions insolubles dans l'éther sont jointes au filtrat. Après évaporation des solvants, le résidu est dissous dans 40 cm$^3$ d'éthanol bouillant. Après filtration de la solution éthanolique chaude et addition d'eau jusqu'à formation d'un trouble persistant, le mélange est laissé au repos. Il se forme un solide qui est isolé par filtration et recristallisé à partir du toluène. Le rendement en produit sec est de 5,69 grammes (65%). L'analyse de ce produit montre qu'il répond à la formule:

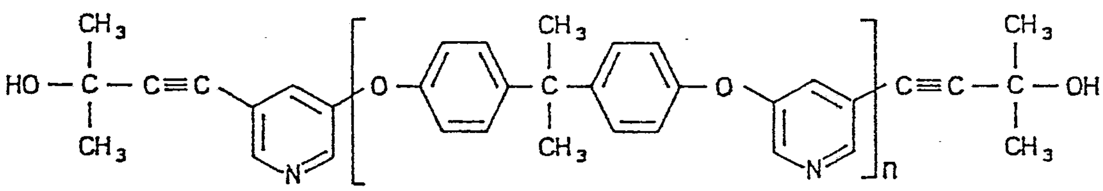

dans laquelle le degré de polycondensation n est égal à 1,05.

Exemple 35

Un mélange de 3,06 grammes des oligomères préparés dans l'exemple 34 principalement constituée de bis((hydroxy-3 méthyl-3 butynyl)-3 pyridyl-5 oxy)-4 phényl) -2,2 propane, 0,34 gramme d'hydroxyde de sodium en poudre et 50 cm$^3$ de toluène est chauffé pendant 210 minutes à 111° C en atmosphère d'azote. Une fraction du solvant, correspondant à environ 12 cm$^3$, est distillée lentement pendant le cours de la réaction afin d'éliminer l'acétone qui se forme. Après refroidissement, la solution est filtrée et lavée à l'eau pour donner, après évaporation du toluène sous pression réduite et un séchage prolongé sous vide, 1,91 grammes d'une huile visqueuse. L'analyse de ce produit montre qu'il répond à la formule:

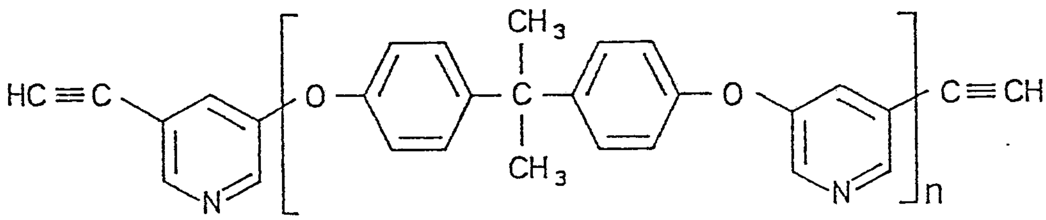

L'analyse de ce produit par chromatographie sur gel perméable montre qu'il est constitué d'oligomères répondant à la formule précédente avec 92% du composé pour lequel le degré de polycondensation n est égal à 1 et 8% de l'oligomère pour lequel n est égal à 2.

Exemple 36

Un mélange de 37,92 grammes (0,16 mole) de dibromo-3,5 pyridine, 18,24 grammes (0,08 mole) de bis(hydroxy-4 phényl)-2,2 propane, 14,35 grammes de carbonate de potassium, 0,88 gramme de chlorure cuivreux, 0,65 gramme d'iodure de potassium, 260 $cm^3$ de N-méthyl pyrrolidinone-2 et 130 $cm^3$ de toluène est chauffé pendant 2 heures à 144° C avec élimination azéotropique de l'eau formée au cours de la réaction. Le toluène distillé est ensuite progressivement enlevé du décanteur de Dean et Stark pendant une durée de 5 heures, la température du mélange réactionnel passant progressivement de 144° C à 156° C. La température est ensuite élevée jusqu'à 180° C en deux heures. Après refroidissement, le mélange réactionnel est filtré et le filtrat est lavé au chloroforme puis refiltré.

Le résidu obtenu par distillation des solvants sous pression réduite est ensuite repris par 150 $cm^3$ d'acétate d'éthyle. La solution est filtrée pour éliminer les impuretés insolubles dans l'acétate d'éthyle. Elle est ensuite percolée sur 30 grammes de gel de silice en grains de 0,08 mm de diamètre moyen. L'évaporation du solvant donne avec un rendement de 46% un produit jaune spongieux. L'analyse de ce produit par chromatographie sur gel perméable montre qu'il est constitué d'oligomères répondant à la formule de l'exemple 33 avec 46% du composé pour lequel le degré de polycondensation n est égal à 1, 51% d'oligomères pour lesquels n est égal et supérieur à 2 et 5% d'un composé non identifié ayant une masse moléculaire apparente de 370 $grammes.mole^{-1}$. Le dosage des atomes de brome qui constituent les groupes terminaux indique que la valeur moyenne du degré de polycondensation n est de 1,99.

Exemple 37

Un mélange de 19,82 grammes du produit dibromé préparé dans l'exemple 36, correspondant à 47,2 milliéquivalents de brome, 4,94 grammes de méthyl-2 butyn-3-ol-2, 66 milligrammes de bis(triphénylphosphine) dichloropalladium, 0,376 gramme de triphénylphosphine, 69 milligrammes d'iodure cuivreux et 88 $cm^3$ de triéthylamine est chauffé pendant 270 minutes au reflux du solvant en atmosphère d'azote. Après refroidissement, le mélange est filtré et lavé à l'eau. Après évaporation des solvants, le résidu gommeux est dissous dans 40 $cm^3$ d'éthanol bouillant. Après filtration de la solution éthanolique chaude et addition d'eau jusqu'à formation d'un trouble persistant, le mélange est laissé au repos. Il se forme une phase visqueuse qui se dépose au fond du récipient. Cette phase est séparée et séchée sous vide. Le rendement en produit sec est de 15,02 grammes (75%). L'analyse de ce produit montre qu'il répond à la formule générale de l'exemple 34 avec un degré de polycondensation moyen n qui est égal à 2.

Une solution de 13,5 grammes de ce produit dans 150 $cm^3$ de toluène est chauffée à 111° C, en présence de 1,4 grammes d'hydroxyde de sodium en poudre, pendant cinq heures. Une fraction du solvant, correspondant à environ 37 $cm^3$, est distillée lentement pendant le cours de la réaction afin d'éliminer l'acétone qui se forme. Après refroidissement, la solution est filtrée et lavée à l'eau pour donner, après évaporation du toluène sous pression réduite et un séchage prolongé sous vide, 8,54 grammes d'une huile visqueuse. L'analyse de ce produit montre qu'il répond à la formule générale de l'exemple 35 dans laquelle le degré de polycondensation n, déterminé par l'analyse élémentaire et par résonance magnétique nucléaire a une valeur moyenne de 2,5.

Exemple 38

En utilisant les conditions expérimentales décrites dans l'exemple 36, on effectue la réaction de polycondensation de la dibromo-3,5 pyridine avec le bis(hydroxy-4 phényl)-2,2 propane en utilisant un rapport molaire de ces deux réactifs de 1,5. Le produit obtenu à la fin de la réaction est un solide qui répond à la formule générale de l'exemple 33. L'analyse de ce produit par chromatographie sur gel perméable montre qu'il est constitué d'oligomères de polyaryloxypyridines terminés par des atomes de brome avec 29% du composé pour lequel le degré de polycondensation n est égal à 1, 67% d'oligomères pour lesquels n est égal ou supérieur à 2 et 4% d'un composé non identifié ayant une masse moléculaire apparente de 370 $grammes.mole^{-1}$. Le dosage de brome indique que la valeur moyenne du degré de polycondensation n est de 3,37.

Exemple 39

Le mélange d'oligomères préparé dans l'exemple 38 est mis en réaction avec le méthyl-2-butyn-3-ol-2-en utilisant les conditions décrites dans l'exemple 37. Cette opération conduit à la substitution des atomes de brome par des groupes acétyléniques protégés avec un rendement de 52%. Le traitement ultérieur de 5,76 grammes de ce dernier produit par 0,5 grammes d'hydroxyde de sodium dans 80 $cm^3$ de toluène donne 4,29 grammes (82%) d'une huile très visqueuse, de couleur marron, qui répond à la formule générale de l'exemple 35dans laquelle le degré de polycondensation moyen n est de 3,3.

Exemple 40

En utilisant les conditions expérimentales décrites dans l'exemple 36, on effectue la réaction de polycondensation de la dibromo-3,5 pyridine avec le bis(hydroxy-4 phényl)-2,2 propane en utilisant un rapport molaire de ces deux réactifs de 1,15. Le produit obtenu à la fin de la réaction, avec un rendement de 42%, est un solide dont l'analyse par chromatographie sur gel perméable montre qu'il est constitué d'oligomères répondant à la formule générale de l'exemple 33 avec 11% du composé pour lequel le degré de polycondensation n est égal à 1 et 89% d'oligomères pour lesquels n est égal ou supérieur à 2.

Ce produit, mis en solution dans du chloroforme, est précipité dans du méthanol pour donner un solide qui

ne contient plus que 6,5% de l'oligomère pour lequel n est égal à 1. Le dosage des atomes de brome et l'analyse élémentaire indiquent que la valeur moyenne du degré de polycondensation n est égale à 11, soit une masse moléculaire moyenne en nombre proche de 3500 grammes.mole$^{-1}$.

Ce mélange de polyaryloxypyridines dibromées est étudié par analyse thermogravimétrique dynamique en atmosphère d'azote avec une vitesse de montée en température de 10°C par minute. Le seuil de décomposition thermique se situe vers 260°C et la perte de poids atteint 1% à 350°C et 5% à 410°C.

Exemple 41

La fraction d'oligomères de polyaryloxypyridines dibromées, solubles dans l'acétate d'éthyle mais insolubles dans l'hexane, isolée dans l'exemple 33 est traitée comme décrit dans l'exemple 39 pour donner, avec un rendement de 40%, un liquide visqueux qui répond à la formule générale de l'exemple 35.

L'analyse de ce produit par chromatographie sur gel perméable montre qu'il est constitué d'oligomères contenant 42% du composé pour lequel le degré de polycondensation n est égal à 1, 33% de l'oligomère pour lequel n est égal à 2 et 24% d'oligomères ayant un degré de polycondensation égal ou superieur à 3. Le dosage de brome, l'analyse élémentaire et la résonance magnétique nucléaire indiquent que le degré moyen de polycondensation a une valeur de 1,7 mais avec une distribution des masses moléculaires élargie par comparaison avec les oligomères décrits dans les exemples 35, 37 et 39.

Exemple 42

Les caractéristiques thermiques des oligomères préparés dans l'exemple 35 sont étudiées au moyen d'un appareil d'analyse calorimétrique différentielle programmé pour une montée en température de 10°C par minute. Les thermogrammes sont tracés entre 20°C et 370°C. Lors de la première montée en température, le thermogramme présente un pic exothermique de polymérisation qui commence à 145°C, passe par un maximum à 242°C et se termine vers 300°C. Aucune autre transition thermique n'est observable avant ce pic de polymérisation. Après refroidissement de la cellule de mesure, une seconde montée en température montre que le pic de polymérisation a disparu et qu'il n'y a aucune variation de la pente du thermogramme qui pourrait indiquer la présence d'une transition vitreuse.

A partir de ces données, on prépare un matériau en polymérisant quelques grammes de la composition d'oligomères de l'exemple 35 dans une coupelle métallique pendant 2 heures à 180°C.

Le réseau obtenu au cours de cette réaction de polymérisation thermique est ensuite étudié par analyse thermogravimétrique dynamique dans l'air avec une vitesse de montée en température de 5°C par minute. Le seuil de dégradation du produit se situe à 420°C et la perte de poids est de 1% à 440°C et de 5% à 450°C.

Exemple 43

Les analyses thermiques décrits dans l'exemple 42 sont effectuées sur les oligomères décrits dans les exemples 37, 39 et 41 avec les résultats suivants :

| Résine de l'exemple | 37 | 39 | 41 |
|---|---|---|---|
| DPn de la résine | 2,5 | 3,3 | 1,7 |
| Début de polymérisation | 140°C | 180°C | 140°C |
| Maximum de l'exotherme | 245°C | 225°C | 237°C |
| Fin de polymérisation | 305°C | 295°C | 310°C |
| Transition vitreuse (au deuxième passage) | 120°C | 123°C | néant |
| 1% de décomposition à l'air | 406°C | 350°C | 415°C |
| 5% de décomposition à l'air | 434°C | 415°C | 460°C |

**Revendications**

1- Compositions d'oligomères de polyaryloxypyridine à terminaisons acétyléniques, caractérisée en ce qu'elle est obtenue par formation d'une composition de polyaryloxypyridine à terminaisons halogénées à partir d'au moins un diphénolate alcalin et d'au moins une dihalogénopyridine, suivie d'une réaction d'éthynylation.

2- Compositions selon la revendication 1, caractérisée en ce qu'elle est représentée par la formule

générale

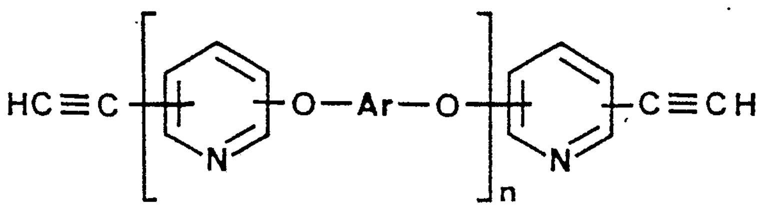

dans laquelle Ar représente un radical aromatique divalent, carbocyclique ou hétérocyclique, formé d'un ou de plusieurs cycles accolés ou reliés entre eux par une liaison simple ou par un atome ou groupement divalent, les deux valences dudit radical aromatique Ar étant situées sur deux atomes de carbone distincts; et n représente le degré moyen de polycondensation.

3- Composition selon la revendication 2, caractérisée en ce que ledit degré moyen de polycondensation a une valeur de 1 à 50.

4- Procédé de préparation d'une composition d'ologimères selon l'une des revendications 1 à 3, caractérisé en ce qu'il comprend

a) la préparation d'une composition de polyaryloxypyridine à terminaisons halogénées par une méthode dans laquelle :

- (i) on prépare un diphénolate alcalin en faisant réagir un diphénol avec un réactif alcalin choisi parmi les métaux alcalins, leurs hydroxydes, leurs carbonates et leurs alcoolates; et

- (ii) on fait réagir le diphénolate alcalin obtenu avec une dihalogénopyridine, ledit diphénolate alcalin étant en défaut moléculaire par rapport à ladite dihalogénopyridine, de manière à obtenir une composition d'oligomères de polyaryloxypyridine à terminaisons halogénées que l'on sépare du milieu réactionnel; et

b) la transformation de ladite composition d'oligomères de polyaryloxypyridine à terminaisons halogénées en une composition de polyaryloxypyridine à terminaisons acétyléniques par une méthode dans laquelle :

- (iii) on fait réagir ladite composition d'oligomères de polyaryloxypyridine à terminaisons halogénées avec au moins un composé acétylénique dont l'un des sites réactifs est protégé par un groupe protecteur, de manière à former des terminaisons acétyléniques substituées; et

- (iv) on transforme lesdites terminaisons acétyléniques substituées en terminaisons acétyléniques libres.

5- Procédé selon la revendication 4, caractérisé en ce que, dans l'étape (a) (i), la proportion molaire du diphénolate alcalin à la dihalogénopyridine est de 1/1,02 à 1/2 et la réaction est effectuée à une température de 50 à 300° C.

6- Procédé selon l'une des revendications 4 et 5, caractérisé en ce que, dans l'étape (b) (iii), ledit composé acétylénique répond à la formule générale

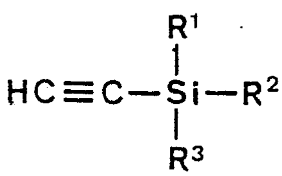

ou à la formule générale

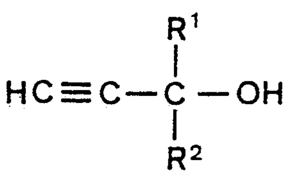

où $R^1$, $R^2$ et $R^3$ représentent chacun un reste monovalent aliphatique ou aromatique de 1 à 13 atomes de carbone.

7- Procédé selon l'une des revendications 4 à 6, caractérisé en ce que dans l'étape (b) (iii) la réaction est effectuée en solution dans un solvant organique, en présence d'un composé basique susceptible de fixer les molécules d'hydracide et d'un système catalytique comprenant un composé du cuivre et un composé du palladium, à une température de 20 à 200° C.

8- Procédé selon la revendication 7, caractérisé en ce que ledit composé du cuivre est un composé

cuivreux et en ce que ledit composé du palladium répond à l'une des formules $Pd(PR_3)_4$, $Pd(PR_3)_2X_2$, $Pd(O_2CR)_2(PR_3)_2$, $Pd(O_2CR)_2$ et $Pd(AsR_3)_2X_2$ dans laquelle X est un atome d'halogène et R représente un groupe alkyle, aralkyle ou aryle.

9- Procédé selon l'une des revendications 4 à 8, caractérisé en ce que le composé acétylénique utilisé dans l'etape (b) (iii) est un composé de formule

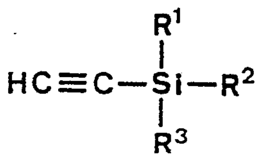

où $R^1$, $R^2$ et $R^3$ représentent chacun un reste monovalent aliphatique ou aromatique de 1 à 13 atomes de carbone; et en ce que dans l'étape (b) (iv) on effectue une réaction de désilylation en solution dans un mono-alcool aliphatique en présence d'une base minérale et à une température de 50 à 150° C.

10- Procédé selon l'une des revendications 4 à 8, caractérisé en ce que le composé acétylénique utilisé dans l'étape (b) (iii) est un composé de formule

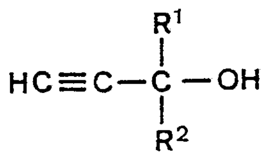

où $R^1$ et $R^3$ représentent chacun un reste monovalent aliphatique ou aromatique de 1 à 13 atomes de carbone; et en ce que dans l'étape (b) (iv) on effectue l'élimination de la cétone de formule $R^1$-CO-$R^2$, en solution dans un solvant organique, en présence d'une base minérale et à une température de 50 à 150° C.

11- Utilisation d'une composition d'oligomères de polyaryloxypyridine à terminaisons acétyléniques dans laquelle ladite composition est chauffée à une température de 100 à 250°C pour former des polymères réticulés.

12- Utilisation selon la revendication 11, caractérisé en ce que le chauffage est poursuivi à une température de 200 à 300° C

13- Polymère réticulé obtenu selon l'une des revendications 11 et 12.

Office européen des brevets

# RAPPORT DE RECHERCHE EUROPEENNE

Numero de la demande

EP 87 40 2209

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-1 527 714 (INSTITUT FRANCAIS DU PETROLE)<br>* Résumé * | 1 | C 08 G 65/48<br>C 08 G 65/40<br>C 08 G 61/10 |
| A | EP-A-0 059 646 (JAPAN SYNTHETIC RUBBER)<br>* Revendications, exemples * | 1 | |
| A | US-A-3 705 131 (V.V. KORSHAK et al.)<br>* Revendications; colonne 3, lignes 1-42 * | 1 | |

DOMAINES TECHNIQUES RECHERCHES (Int. Cl.4)

C 08 G
C 07 D

Le présent rapport a été établi pour toutes les revendications

| Lieu de la recherche | Date d'achèvement de la recherche | Examinateur |
|---|---|---|
| LA HAYE | 25-01-1988 | DERAEDT G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)